# EUROPÄISCHE PATENTSCHRIFT

(11) **EP 3 964 491 B1**
(45) Veröffentlichungstag und Bekanntmachung des Hinweises auf die Patenterteilung: **24.05.2023**
(21) Anmeldenummer: 20194598.7
(22) Anmeldetag: 04.09.2020
(51) Int. Cl.: C07C 2/08

(54) **VERFAHREN ZUR OLIGOMERISIERUNG VON ISOBUTEN**
METHOD FOR OLIGOMERIZING ISOBUTENE
PROCÉDÉ D'OLIGOMÉRISATION D'ISOBUTÈNE

(43) Veröffentlichungstag der Anmeldung: 09.03.2022
(73) Patentinhaber: Evonik Operations GmbH, 45128 Essen (DE)
(72) Erfinder: PEITZ, Stephan, 45739 Oer-Erkenschwick (DE); STOCHNIOL, Guido, 45721 Haltem am See (DE); KNOSSALLA, Johannes, 46514 Schermbeck (DE)
(74) Vertreter: Evonik Patent Association

(56) Entgegenhaltungen:
- WO-A1-01/51435
- GB-A- 959 756
- US-A1- 2014 128 652

## Beschreibung

Die vorliegende Erfindung betrifft ein Verfahren zur Oligomerisierung von Isobuten durch Umsetzung eines isobutenhaltigen Kohlenwasserstoffstroms an einem sauren Katalysator in mindestens einer Reaktionsstufe, wobei ein bestimmtes Verhältnis von Recycle zu Feed eingesetzt wird.

Die Oligomerisierung von Isobuten ist ein bekanntes großtechnisches Verfahren zur Herstellung von Diisobuten. Diisobuten ist dabei die technische Bezeichnung für ein Gemisch aus 2,4,4-Trimethylpent-1-en und 2,4,4-Trimethylpent-2-en, welches in der Synthese, beispielsweise der Hydroformylierung zum Aldehyd, oder nach Hydrierung als Kraftstoffkomponente eingesetzt wird. Bekannte Maßnahmen zur technischen Verbesserung dieses Verfahrens sind beispielsweise bereits in der EP 1 388 528 A1 und der US 2014/128652 A1 beschrieben.

Trotz der bekannten Verfahren besteht ein kontinuierlicher Bedarf an Verfahrensverbesserungen, insbesondere im Hinblick auf die Selektivität zu 2,4,4-Trimethylpent-1-en. Für die Hydroformylierung von Diisobuten ist es nämlich von Vorteil, wenn überwiegend 2,4,4-Trimethylpenten-1 im Diisobuten vorliegt. Es wurde nun überraschend gefunden, dass die Selektivität zu 2,4,4-Trimethylpent-1-en in Abhängigkeit des Recycle-zu-Feed-Verhältnisses gesteigert werden kann.

Die zugrundeliegende Aufgabe der vorliegenden Erfindung, die in der Bereitstellung eines verbesserten Verfahrens zur Oligomerierung von Isobuten mit einer höheren Selektivität zu 2,4,4-Trimethylpenten-1 im gebildeten Diisobuten bestand, konnte durch das anspruchsgemäße Verfahren gelöst werden. Bevorzugte Ausgestaltungen des Verfahrens sind in den Unteransprüchen angegeben.

Das erfindungsgemäße Verfahren ist demnach ein Verfahren zur Herstellung von Diisobuten durch Oligomerisierung von Isobuten an einem sauren Katalysator in mindestens einer Reaktionsstufe, die jeweils mindestens einen Reaktor und mindestens eine Destillationskolonne umfasst, wobei ein isobutenhaltiger Einsatzstrom, der einen isobutenhaltigen Kohlenwasserstoffstrom als Feed und einen zurückgeführten Strom als Recycle umfasst, in dem mindestens einen Reaktor der mindestens einen Reaktionsstufe unter Erhalt einer Produktmischung, die zumindest das gebildete Diisobuten mit einem Anteil an 2,4,4-Trimethylpent-1-en von mindestens 75 Mol%, vorzugsweise mindestens 76,5 Mol%, besonders bevorzugt mindestens 78 Mol% und nicht umgesetztes Isobuten enthält, umgesetzt und die erhaltene Produktmischung der mindestens einen Destillationskolonne zugeführt wird,
am Kopf der Destillationskolonne ein, bezogen auf die erhaltene Produktmischung, an dem gebildeten Diisobuten abgereicherter Restkohlenwasserstoffstrom abgenommen und zumindest teilweise als Recycle zum mindestens einen Reaktor zurückgeführt wird, wobei das Verhältnis von Recycle zu Feed mindestens 4 beträgt.

Es wurde überraschend festgestellt, dass die Selektivität der Oligomerisierungsreaktion zu 2,4,4-Trimethylpent-1-en aufgrund des Verhältnisses von Recycle zu Feed im isobutenhaltigen Einsatzstrom von mindestens 4 erhöht werden kann. Vorzugsweise beträgt das Verhältnis von Recycle zu Feed mindestens 5, besonders bevorzugt mindestens 6. Dadurch lassen sich noch bessere Selektivitäten zu 2,4,4-Trimethylpent-1-en erzielen.

Der bei der erfindungsgemäßen Oligomerisierung von Isobuten zur Herstellung von Diisobuten im ersten Reaktor und - sofern vorhanden - bei jedem zum ersten Reaktor parallel geschalteten Reaktor jeder vorhandenen Reaktionsstufe eingesetzte isobutenhaltige Einsatzstrom umfasst sowohl einen isobutenhaltigen Kohlenwasserstoffstrom als Feed (Frischfeed) als auch einen aus der der Oligomerisierung nachfolgenden Destillation zurückgeführten Strom als Recycle. Bevorzugt besteht dieser eingesetzte isobutenhaltige Einsatzstrom aus einem isobutenhaltigen Kohlenwasserstoffstrom als Feed (Frischfeed), einem zurückgeführten Strom als Recycle und optional einem Verdünnungsstrom aus inerten Alkanen. Sofern beim erfindungsgemäßen Verfahren zum ersten Reaktor jeder vorhandenen Reaktionsstufe einer oder mehrere in Reihe geschaltete Reaktoren vorliegen, werden diese nur mit dem Austrag aus dem vorherigen Reaktor gespeist ohne zusätzlich den Recycle zuzuführen.

Als isobutenhaltiger Strom, der als Feed beim erfindungsgemäßen Verfahren eingesetzt wird, können grundsätzliche alle isobutenhaltigen Kohlenwasserstoffgemische eingesetzt werden, die Isobuten in einer Menge enthalten, die es erlaubt das Verfahren wirtschaftlich zu betreiben. Bevorzugt werden Mischungen von Isobuten und weiteren C4-Kohlenwasserstoffen, also C4-Kohlenwasserstoffströme eingesetzt. Im Sinne der vorliegenden Erfindung gilt auch der Austrag aus einem vorherigen Oligomerisierungsreaktor als isobutenhaltiger Strom. Technische Gemische, die Isobuten enthalten, sind beispielsweise Leichtbenzinfraktionen aus Raffinerien, C4-Fraktionen aus Crackern (beispielsweise Steamcracker, Hydrocracker, Katcracker), Gemische aus Fischer-Tropsch-Synthesen, Gemische aus der Dehydrierung von Butanen, Gemische aus der Skelettisomerisierung linearer Butene und Gemische, entstanden durch Metathese von Olefinen.

In einer besonders bevorzugten Ausführungsform der vorliegenden Erfindung wird ein reiner Isobutenstrom als isobutenhaltiger Kohlenwasserstoffstrom, also als Feed, eingesetzt. Der Begriff "rein" ist in diesem Zusammenhang so zu verstehen, dass ein reiner Isobutenstrom mindestens 98 Gew.-% Isobuten, bezogen auf die Gesamtmasse des Stroms, enthält. Die Isobutenkonzentration am Eingang der ersten Reaktionsstufe bzw. des ersten Reaktors der ersten Reaktionsstufe, also die Isobutenkonzentration im eingesetzten isobutenhaltigen Einsatzstroms, soll vorzugsweise 90%, weiterhin bevorzugt 87%, besonders bevorzugt 85% nicht überschreiten.

Um die Isobutenkonzentration zur Begrenzung eines Temperaturanstiegs im Reaktor zu begrenzen kann der isobutenhaltige Kohlenwasserstoffstrom mit einer definierten Menge eines Löse- oder Verdünnungsmittels versetzt werden. Als Löse- oder Verdünnungsmittel eignen sich inerte Substanzen wie Alkane, vorzugsweise Isobutan.

Der Gesamtzulauf des isobutenhaltigen Einsatzstroms der ersten Reaktionsstufe bzw. des ersten Reaktors der ersten Reaktionsstufe kann in weiten Bereichen variieren. Dabei kommt es auf den tatsächlichen Aufbau (ein oder mehrere Reaktoren, etc.) und die Größe von Reaktor(en) und Destillationskolonne(n) an. In einer bevorzugten Ausführungsform der vorliegenden Erfindung wird die erste Reaktionsstufe bzw. der erste Reaktor der ersten Reaktionsstufe oder alle in Reihe geschalteten Reaktoren der ersten Reaktionsstufe mit einem Gesamtzulauf des isobutenhaltigen Einsatzstroms von mindestens 2,5 t pro t Katalysator und Stunde (h) (Einheit t/(t*h)), vorzugsweise mindestens 3 t/(t*h) und besonders bevorzugt mindestens 3,5 t/(t*h) befahren. Der Gesamtzulauf ist die Menge an Feed bezogen auf die Masse des vorliegenden Katalysators pro Zeiteinheit (Stunde). Diese wird immer auf die gesamte Katalysatormasse von in Reihe geschalteten Reaktoren bezogen. Liegt nur ein Reaktor vor, ist die Katalysatormasse die Masse des Katalysators in dem einem Reaktor. Liegen zwei oder mehr in Reihe geschaltete Reaktoren vor ist die Katalysatormasse die Gesamtsumme der Katalysatormassen aus den zwei oder mehr in Reihe geschalteten Reaktoren.

Die erfindungsgemäße Oligomerisierung von Isobuten wird in mindestens einer Reaktionsstufe durchgeführt. Es kann vorteilhaft zwei oder mehr Reaktionsstufen zu verwenden, für die erfindungsgemäße Oligomerisierung von Isobuten ist es aber bevorzugt, dass nur eine Reaktionsstufe vorliegt. Das ist anlagentechnisch einfacher zu realisieren und potentiell mit weniger Kostenaufwand verbunden.

Eine Reaktionsstufe umfasst im Sinne der vorliegenden Erfindung mindestens einen Reaktor und mindestens eine Destillationskolonne. Es können auch mehrere Reaktoren und/oder mehrere Destillationskolonnen vorliegen. Ist mehr als eine Reaktionsstufe vorhanden, kann die Anzahl der Reaktoren und/oder Destillationskolonnen bei allen Reaktionsstufen gleich oder unterschiedlich sein. Es ist vorliegend bevorzugt, dass eine Reaktionsstufe mindestens zwei Reaktoren umfasst, die seriell oder parallel zueinander verschaltet sind. Liegen die beiden Reaktoren parallel geschaltet vor, werden beiden Reaktoren Frischfeed und Recycle jeweils im erfindungsgemäßen Verhältnis zugeführt. Liegen die Reaktoren in Reihe geschaltet vor, wird dem zweiten Reaktor die Produktmischung aus dem ersten Reaktor zugeführt.

Die Oligomerisierung von Isobuten kann gemäß der vorliegenden Erfindung chargenweise oder bevorzugt kontinuierlich durchgeführt werden. Geeignete Reaktoren für die kontinuierliche Verfahrensführung sind Strömungsreaktoren, beispielsweise Festbettreaktoren, Rohrbündelreaktoren, kontinuierliche Rührkesselreaktoren, Schlaufenreaktoren oder eine Kombination davon. Für die chargenweise Verfahrensführung eignen sich Rührkesselreaktoren. Die im vorliegenden Verfahren eingesetzten Reaktoren können adiabatisch, polytrop oder nahezu isotherm, d. h. unter Verwendung eines Kühlmittels, mit dem der Reaktor gekühlt wird, betrieben werden. Nahezu isotherm bedeutet, dass die Temperatur an einer beliebigen Stelle im Reaktor maximal um 15 K höher ist als die Temperatur am Reaktoreingang.

Die Oligomerisierung von Isobuten erfolgt durch Inkontaktbringen des isobutenhaltigen Einsatzstroms mit einem sauren Katalysator. Die Temperatur bei der Oligomerisierung liegt vorzugsweise zwischen 5 und 160 °C, besonders bevorzugt zwischen 30 und 110°C, ganz besonders bevorzugt zwischen 40 und 90°C.

Die erfindungsgemäße Oligomerisierung kann weiterhin bei einem Druck gleich oder über dem Dampfdruck des Einsatzstroms bei der jeweiligen Reaktionstemperatur durchgeführt werden, vorzugsweise bei einem Druck unter 40 bar. Der isobutenhaltige Einsatzstrom sollte während der Oligomerisation ganz oder teilweise in flüssiger Phase vorliegen. In einer bevorzugten Ausführungsform findet die Oligomerisierung in flüssiger Phase statt. Die Einstellung von Druck und Temperatur, um eine Flüssigphasenreaktion zu erhalten, sind dem Fachmann bekannt und ergeben sich auf Basis der oben genannten Bereiche.

Der bei der Oligomerisierung von Isobuten eingesetzte Katalysator ist ein saurer Katalysator. Typische saure Katalysatoren für die Oligomerisierung von Isobuten zu Diisobuten sind dem Fachmann bekannt. In einer bevorzugten Ausführungsform wird als saurer Katalysator ein saures lonenaustauscherharz eingesetzt, bei dem ein Teil der aciden Protonen gegen Metallionen ausgetauscht wurden.

Bei der erfindungsgemäßen Oligomerisierung werden weiterhin bevorzugt feste sulfonierte lonenaustauscherharze eingesetzt, bei denen insbesondere 0,1 bis 70 % der aciden Protonen, bevorzugt 30 bis 65 % der aciden Protonen der Sulfonsäuregruppen gegen Metallionen ausgetauscht worden sind. Als Metallionen, die die Protonen ersetzen, eignen sich Ionen von Alkalimetallen, Erdalkalimetallen, Chrom, Mangan, Eisen, Kobalt, Nickel, Zink und Aluminium sowie Ionen der Lanthanidgruppe (Seltene Erden). Bevorzugt werden dafür Alkalimetallionen, insbesondere Natriumionen eingesetzt. Es ist auch möglich, dass der Austausch mit zwei oder mehr unterschiedlichen Metallionen durchgeführt wird.

Geeignete lonenaustauscherharze sind beispielsweise solche, die durch Sulfonierung von Phenol/Aldehyd-Kondensaten oder von Cooligomeren von aromatischen Vinylverbindungen hergestellt werden. Beispiele für aromatische Vinylverbindungen zur Herstellung der Cooligomere sind Styrol, Vinyltoluol, Vinylnaphthalin, Vinylethylbenzol, Methylstyrol, Vinylchlorbenzol, Vinylxylol und Divinylbenzol. Insbesondere werden die Cooligomere, die durch Umsetzung von Styrol mit Divinylbenzol entstehen, als Vorstufe für die Herstellung der hier bevorzugten sulfonierten lonenaustauscherharze verwendet. Die Eigenschaften dieser Harze, insbesondere spezifische Oberfläche, Porosität, Stabilität, Quellung bzw. Schrumpfung und Austauschkapazität, können durch den Herstellprozess variiert werden. Die Harze können gelförmig, makroporös oder schwammförmig hergestellt werden.

Die Korngröße des erfindungsgemäß als saurer Katalysator eingesetzten Ionenaustauscherharzes liegt bevorzugt zwischen 500 µm und 1500 µm, vorzugsweise zwischen 600 µm und 1000 µm. Die Bestimmung der Korngröße kann mittels Laserlichtbeugung nach ISO 13320:2020-01. erfolgen. Die Korngrößenverteilung kann enger oder weiter gewählt werden. So können beispielsweise lonenaustauscherharze mit sehr einheitlicher Korngröße (monodisperse Harze) eingesetzt werden. Es können aber auch lonenaustauscherharze eingesetzt werden, die als Formkörper, wie beispielsweise Zylinder, Ringe oder Kugeln, vorliegen. Werden bei dem erfindungsgemäßen Verfahren mehrere Reaktoren eingesetzt, können diese mit lonenaustauscherharzen gleicher oder unterschiedlicher Korngröße bzw. mit gleicher oder unterschiedlicher Korngrößenverteilung oder in Form verschiedener Formkörper gefüllt sein.

Die erfindungsgemäß bevorzugten lonenaustauscherharze können nach verschiedenen dem Fachmann bekannten Verfahren hergestellt werden. Liegt das lonenaustauscherharz in der H-Form vor, können Protonen gegen Metallionen ausgetauscht werden. Liegt das Harz als Metallsalz vor, können Metallionen mit Hilfe von Säuren durch Protonen ersetzt werden. Prinzipiell kann dieser lonenaustausch sowohl in organischer als in wässriger Suspension erfolgen. Ein einfaches Verfahren ist die Aufschlämmung des Ionenaustauscherharzes in der H⁺-Form mit ausreichend Flüssigkeit (das ca. Ein- bis Zehnfache des Eigenvolumens des Ionenaustauscherharzes) zur Herstellung einer rührbaren Suspension. Zu dieser Suspension wird dann eine Lösung, die die gewünschten Ionen enthält, zudosiert. Der lonenaustausch erfolgt vorzugsweise im Temperaturbereich von 10 bis 100 °C, besonders bevorzugt bei 20 bis 40 °C. Nach erfolgtem lonenaustausch wird das lonenaustauscherharz gewaschen und getrocknet. Die Trocknung kann im Vakuum oder im Inertgasstrom, beispielsweise im Stickstoffstrom, erfolgen. Die Trockentemperaturen liegen typischerweise zwischen 10 und 120 °C.

Ein bevorzugter Weg zur Herstellung der im erfindungsgemäßen Verfahren verwendeten Katalysatoren ist der Austausch von Protonen gegen Metallionen in wässriger Phase, Waschen des teilausgetauschten lonentauscherharzes mit Wasser und eine anschließende Trocknung.

Die Ionen, mit denen das Harz beladen werden soll, können als Lösungen von Hydroxiden, oder Salzen organischer oder anorganischer Säuren vorliegen. Bei Salzen mehrbasiger Säuren können auch saure Salze eingesetzt werden. Ebenfalls können Verbindungen mit anderen organischen Resten wie beispielsweise Alkoholate oder Acetylacetonate verwendet werden. Als Quelle für die Metallionen werden bevorzugt Metallhydroxide und Salze anorganischer Säuren eingesetzt. Ganz besonders bevorzugt ist der Einsatz der Alkalimetallhydroxide, z. B. Natriumhydroxid, Alkalimetallhalogenide, z. B. Natriumchlorid, Alkalimetallsulfate, z. B. Natriumsulfat, Alkalimetallnitrate, z. B. Natriumnitrat, Erdalkalimetallhydroxide und Erdalkalimetallnitrate.

Nach dem oben beschriebenen Vorgehen können je nach Austauschgrad, lonenart und Harz Katalysatoren unterschiedlicher Aktivität und Selektivität hergestellt werden.

Ein Reaktor in dem erfindungsgemäßen Verfahren kann ein Gemisch von Harzen unterschiedlicher Reaktivität enthalten. Ebenso ist es möglich, dass ein Reaktor Katalysatoren mit unterschiedlicher Aktivität, in Schichten angeordnet, enthält. Wird mehr als ein Reaktor verwendet, können die einzelnen Reaktoren mit Katalysatoren gleicher oder unterschiedlicher Aktivität(en) gefüllt sein.

Bei der erfindungsgemäßen Oligomerisierung wird in dem mindestens einen Reaktor eine Produktmischung erhalten, die zumindest das gebildete Diisobuten mit einem Anteil an 2,4,4-Trimethylpent-1-en von mindestens 75 Mol%, vorzugsweise mindestens 76,5 Mol%, besonders bevorzugt mindestens 78 Mol% und nicht umgesetztes Isobuten enthält. Zusätzlich kann die Produktmischung Isobutan enthalten.

Die Produktmischung wird anschließend der mindestens einen Destillationskolonne der Reaktionsstufe zugeführt, um die Produkte von Leichtsiedern abzutrennen. Die Destillation wird so durchgeführt, dass zumindest die Leichtsieder, d. h. nicht umgesetztes Isobuten und - sofern vorhanden - Isobutan, am Kopf der mindestens einen Destillationskolonne als Restkohlenwasserstoffstrom abgenommen werden. Dieser Restkohlenwasserstoffstrom wird teilweise oder vollständig als Recycle zum mindestens einen Reaktor zurückgeführt. Bei vollständiger Rückführung des Restkohlenwasserstoffstroms der mindestes einen Destillationskolonne lässt sich ein 100%iger Umsatz an Isobuten erzielen. Demnach wird nur so viel Masse an Isobuten in dem isobutenhaltigen Kohlenwasserstoffstrom zu dem mindestens einen Reaktor zugeführt wie Masse an Produkt im Sumpf der mindestens einen Destillationskolonne anfällt.

Die Destillation in der mindestens einen Destillationskolonne wird vorzugsweise bei einem Druck zwischen 2 und 9 bar ü (bar Überdruck), bevorzugt zwischen 3 und 8 bar ü, besonders zwischen 4 und 7 bar ü durchgeführt. Die Temperatur bei der Destillation beträgt im Sumpf der Kolonne vorzugsweise 100 °C bis 220 °C, weiterhin bevorzugt 120 °C bis 210 °C, besonders bevorzugt 150 °C bis 200 °C. In einer besonders bevorzugten Ausführungsform der vorliegenden Erfindung wird die Destillation in der mindestens einen Destillationskolonne mit Rücklauf (Teil des gesamten Destillatstroms (Brüden), der in den Kopf der Kolonne zurückgeführt wird) betrieben. Das Rücklaufverhältnis (Verhältnis von Brüden zum Rücklauf) beträgt vorzugsweise 0,001 bis 2, weiterhin bevorzugt 0,005 bis 1,5 und besonders bevorzugt 0,01 bis 1.

Im Sumpf der Destillationskolonne fällt eine Mischung der entstandenen Oligomere an, wobei die im Sumpf anfallende Mischung vorzugsweise zumindest 80 Gew.-% Diisobuten, besonders bevorzugt mindestens 86 Gew.-% Diisobuten, ganz besonders bevorzugt mindestens 92 Gew.-% Diisobuten umfasst. Ein Teil der Mischung können Triisobutene oder höhere Oligomere sein, insbesondere sind dies aber die Triisobutene.

In einer besonders bevorzugten Ausführungsform umfasst die Reaktionsstufe für das erfindungsgemäße Verfahren mindestens zwei Destillationskolonnen, die vorzugsweise in Reihe geschaltet vorliegen. Die zweite Destillationskolonne wird bei der bevorzugten Reihenschaltung aus dem Sumpf der ersten Destillationskolonne gespeist. Die zweite Destillationskolonne kann dazu verwendet werden, die in der ersten Destillation erhaltene Mischung der entstandenen Oligomere aufzutrennen, um das Diisobuten von eventuell vorhandenen höheren Oligomeren, z.B. Triisobuten, abzutrennen.

In der zweiten Destillationskolonne wird der Druck dazu vorzugsweise auf 0,1 bis 5 bar a (bar absolut), weiterhin bevorzugt auf 0,2 bis 3 bar a und besonders bevorzugt auf 0,3 bis 1,5 bar a eingestellt. Die Temperatur im Sumpf der zweiten Destillationskolonne beträgt vorzugsweise 30 bis 180 °C, weiterhin bevorzugt 45 bis 170 °C und besonders bevorzugt 60 bis 160 °C. Das Rücklaufverhältnis in der zweiten Destillationskolonne beträgt vorzugsweise 0 bis 2, weiterhin bevorzugt 0,01 bis 1,8 und besonders bevorzugt 0,05 bis 1,5.

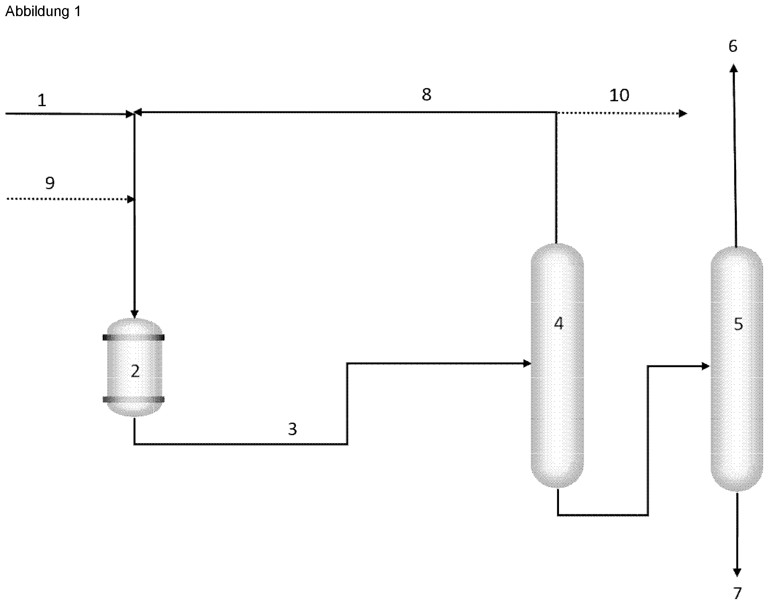

Die vorliegende Erfindung wird nachfolgend anhand der Abbildungen 1 bis 4 erläutert. Es sei darauf hingewiesen, dass diese Abbildungen spezielle Ausführungsformen darstellen und der Erklärung dienen, den Erfindungsgegenstand aber nicht einschränken.

Abbildung 1 zeigt eine Ausführungsform des erfindungsgemäßen Verfahrens mit einem Reaktor (2) in einer Reaktionsstufe mit zwei Destillationskolonnen (4, 5). Dabei wird ein isobutenhaltiger Einsatzstrom aus einem isobutenhaltigen Kohlenwasserstoffstrom (Feed) (1) und einem aus der ersten Destillationskolonne (4) zurückgeführten Strom (Recycle) (8) zum Reaktor (2) geführt, um das enthaltene Isobuten zu oligomerisieren. Das Verhältnis von Recycle zu Feed im isobutenhaltigen Einsatzstrom beträgt erfindungsgemäß mindestens 4, vorzugsweise mindestens 5, besonders bevorzugt mindestens 6. Der Austrag (3) aus dem Reaktor (2), der eine Mischung aus zumindest nicht umgesetztem Isobuten und Isobutenoligomeren ist, wird in der ersten Destillationskolonne (4) von Leichtsiedern (Isobuten und ggf. Isobutan) befreit, die über Kopf der Destillationskolonne (4) abgenommen und zum Reaktor (2) zurückgeführt werden. Der Sumpf der Destillationskolonne (4) besteht im Wesentlichen aus Diisobuten und höheren Oligomeren, die in der nachfolgenden Destillationskolonne (5) voneinander getrennt werden. Das Diisobuten (6) geht über Kopf und die höheren Oligomere (7) fallen im Sumpf an. In der Abbildung sind zwei optionale Ausgestaltungen angedeutet, nämlich die zusätzliche Zufuhr von inerten Alkanen (9) und das Ausschleusen eines Teils der Leichtsieder (10).

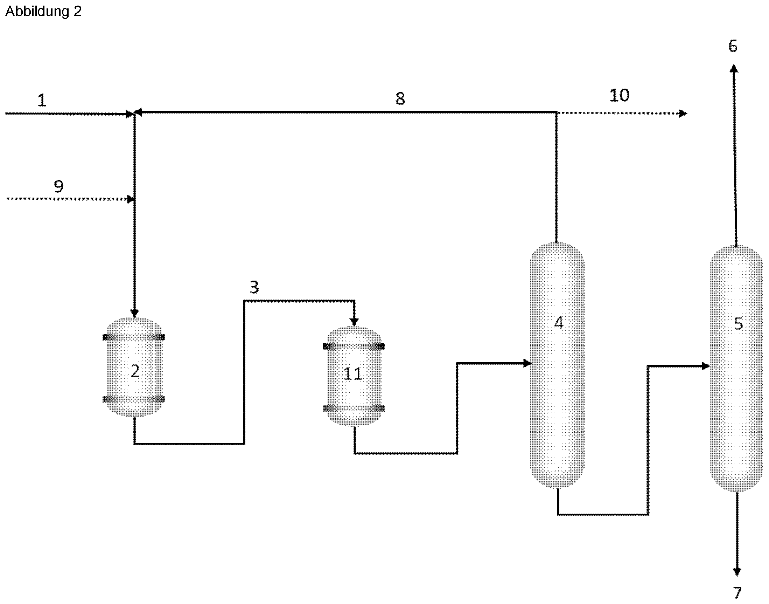

Im Unterschied zur Abbildung 1 weist die in Abbildung 2 gezeigte Ausführungsform zwei Reaktoren (2, 11) auf, die in Reihe geschaltet sind. Ein isobutenhaltiger Einsatzstrom aus einem isobutenhaltigen Kohlenwasserstoffstrom (Feed) (1) und einem aus der ersten Destillationskolonne (4) zurückgeführten Strom (Recycle) (8) wird dabei zum ersten Reaktor (2) geführt, um das enthaltene Isobuten zu oligomerisieren. Das Verhältnis von Recycle zu Feed im isobutenhaltigen Einsatzstrom beträgt erfindungsgemäß mindestens 4, vorzugsweise mindestens 5, besonders bevorzugt mindestens 6. Der Austrag (3) aus dem ersten Reaktor (2), der eine Mischung aus zumindest nicht umgesetztem Isobuten und Isobutenoligomeren ist, wird dann zu einem zweiten Reaktor (11) geleitet, um das noch vorhandene Isobuten zu oligomerisieren. Der Austrag aus dem zweiten Reaktor wird anschließend in der ersten Destillationskolonne (4) von Leichtsiedern (Isobuten und ggf. Isobutan) befreit, die über Kopf der Destillationskolonne (4) abgenommen und zum ersten Reaktor (2) zurückgeführt werden. Der Sumpf der Destillationskolonne (4) besteht im Wesentlichen aus Diisobuten und höheren Oligomeren, die in der nachfolgenden Destillationskolonne (5) voneinander getrennt werden. Das Diisobuten (6) geht über Kopf und die höheren Oligomere (7) fallen im Sumpf an. In der Abbildung sind zudem zwei optionale Ausgestaltungen angedeutet, nämlich die zusätzliche Zufuhr von inerten Alkanen (9) und das Ausschleusen eines Teils der Leichtsieder (10).

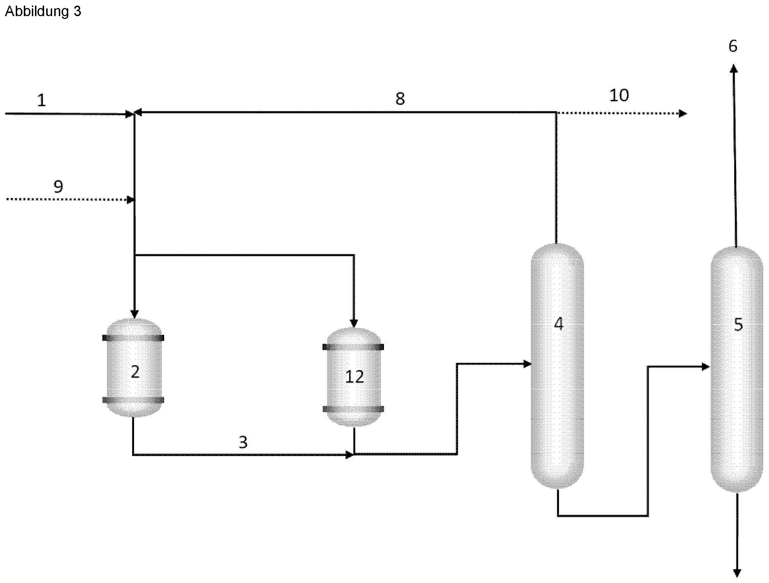

Die Ausführungsform nach Abbildung 3 weist ebenfalls zwei Reaktoren (2, 12), die jedoch parallel geschaltet vorliegen. Ein isobutenhaltiger Einsatzstrom aus einem isobutenhaltigen Kohlenwasserstoffstrom (Feed) (1) und einem aus der ersten Destillationskolonne (4) zurückgeführten Strom (Recycle) (8) wird dabei zum ersten Reaktor (2) und zum zweiten Reaktor (12) geführt, um das enthaltene Isobuten zu oligomerisieren. Das Verhältnis von Recycle zu Feed im isobutenhaltigen Einsatzstrom beträgt erfindungsgemäß mindestens 4, vorzugsweise mindestens 5, besonders bevorzugt mindestens 6. Der Austrag (3) aus dem ersten Reaktor (2) und der Austrag aus dem zweiten Reaktor (12), die jeweils eine Mischung aus zumindest nicht umgesetztem Isobuten und Isobutenoligomeren sind, werden dann in der ersten Destillationskolonne (4) von Leichtsiedern (Isobuten und ggf. Isobutan) befreit, die über Kopf der Destillationskolonne (4) abgenommen und zum ersten und zweiten Reaktor (2, 12) zurückgeführt werden. Der Sumpf der Destillationskolonne (4) besteht im Wesentlichen aus Diisobuten und höheren Oligomeren, die in der nachfolgenden Destillationskolonne (5) voneinander getrennt werden. Das Diisobuten (6) geht über Kopf und die höheren Oligomere (7) fallen im Sumpf an. In der Abbildung sind zudem zwei optionale Ausgestaltungen angedeutet, nämlich die zusätzliche Zufuhr von inerten Alkanen (9) und das Ausschleusen eines Teils der Leichtsieder (10).

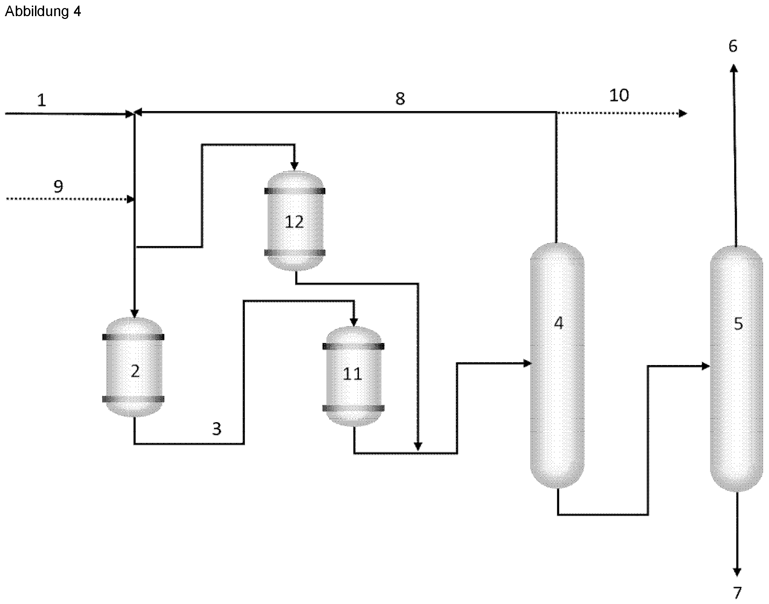

Abbildung 4 veranschaulicht eine Ausführungsform der vorliegenden Erfindung mit 3 Reaktoren (2, 11, 12), wobei jeweils ein Reaktor parallel und ein Reaktor in Reihe geschaltet zum ersten Reaktor vorliegen. Ein isobutenhaltiger Einsatzstrom aus einem isobutenhaltigen Kohlenwasserstoffstrom (Feed) (1) und einem aus der ersten Destillationskolonne (4) zurückgeführten Strom (Recycle) (8) wird dabei zum ersten Reaktor (2) und zum parallel geschalteten zweiten Reaktor (12) geführt, um das enthaltene Isobuten zu oligomerisieren. Das Verhältnis von Recycle zu Feed im isobutenhaltigen Einsatzstrom beträgt erfindungsgemäß mindestens 4, vorzugsweise mindestens 5, besonders bevorzugt mindestens 6. Der Austrag (3) aus dem ersten Reaktor (2) wird zu einem in Reihe geschalteten dritten Reaktor (11) geleitet, um das noch vorhandene Isobuten zu oligomerisieren. Der Austrag aus dem dritten Reaktor (11) und der Austrag aus dem zweiten Reaktor (12), die jeweils eine Mischung aus zumindest nicht umgesetztem Isobuten und Isobutenoligomeren sind, werden dann in der ersten Destillationskolonne (4) von Leichtsiedern (Isobuten und ggf. Isobutan) befreit, die über Kopf der Destillationskolonne (4) abgenommen und zum ersten und zweiten Reaktor (2, 12) zurückgeführt werden. Der Sumpf der Destillationskolonne (4) besteht im Wesentlichen aus Diisobuten und höheren Oligomeren, die in der nachfolgenden Destillationskolonne (5) voneinander getrennt werden. Das Diisobuten (6) geht über Kopf und die höheren Oligomere (7) fallen im Sumpf an. In der Abbildung sind zudem zwei optionale Ausgestaltungen angedeutet, nämlich die zusätzliche Zufuhr von inerten Alkanen (9) und das Ausschleusen eines Teils der Leichtsieder (10).

## Patentansprüche

1. Verfahren zur Herstellung von Diisobuten durch Oligomerisierung von Isobuten an einem sauren Katalysator in mindestens einer Reaktionsstufe, die jeweils mindestens einen Reaktor und mindestens eine Destillationskolonne umfasst, wobei
ein isobutenhaltiger Einsatzstrom, der einen isobutenhaltigen Kohlenwasserstoffstrom als Feed und einen zurückgeführten Strom als Recycle umfasst, in dem mindestens einen Reaktor der mindestens einen Reaktionsstufe unter Erhalt einer Produktmischung, die zumindest das gebildete Diisobuten mit einem Anteil an 2,4,4-Trimethylpent-1-en von mindestens 75 Mol%, vorzugsweise mindestens 76,5 Mol%, besonders bevorzugt mindestens 78 Mol% und nicht umgesetztes Isobuten enthält, umgesetzt und die erhaltene Produktmischung der mindestens einen Destillationskolonne zugeführt wird,
am Kopf der Destillationskolonne ein, bezogen auf die erhaltene Produktmischung, an dem gebildeten Diisobuten abgereichter Restkohlenwasserstoffstrom abgenommen und zumindest teilweise als Recycle zum mindestens einen Reaktor zurückgeführt wird, **dadurch gekennzeichnet, dass**
das Verhältnis von Recycle zu Feed mindestens
4 beträgt.

2. Verfahren nach Anspruch 1, wobei das Verhältnis von Recycle zu Feed mindestens 5 beträgt.

3. Verfahren nach Anspruch 2, wobei das Verhältnis von Recycle zu Feed mindestens 6 beträgt.

4. Verfahren nach einem der Ansprüche 1 bis 3, wobei der isobutenhaltige Kohlenwasserstoffstrom ein C4-Kohlenwasserstoffstrom ist.

5. Verfahren nach einem der Ansprüche 1 bis 3, wobei der isobutenhaltige Kohlenwasserstoffstroms ein reiner Isobutenstrom ist.

6. Verfahren nach einem der Ansprüche 1 bis 5, wobei der am Kopf der Kolonne abgenommene Restkohlenwasserstoffstrom vollständig zum mindestens einen Reaktor zurückgeführt wird.

7. Verfahren nach einem der Ansprüche 1 bis 6, wobei eine Reaktionsstufe mindestens zwei Reaktoren umfasst, die seriell oder parallel zueinander geschaltet sind.

8. Verfahren nach einem der Ansprüche 1 bis 7, wobei die Oligomerisierung in flüssiger Phase durchgeführt wird.

9. Verfahren nach einem der Ansprüche 1 bis 8, wobei der saure Katalysator ein saures lonenaustauscherharz ist, bei dem ein Teil der aciden Protonen gegen Metallionen ausgetauscht wurden.

10. Verfahren nach Anspruch 9, wobei 0,1 bis 70 % der aciden Protonen, bevorzugt 30 bis 65 % der aciden Protonen des Ionenaustauschers gegen Metallionen ausgetauscht wurden.

11. Verfahren nach Anspruch 9 oder 10, wobei die Metallionen Ionen der Alkalimetalle, Erdalkalimetalle und/oder Seltenen Erden sind.

12. Verfahren nach einem der Ansprüche 1 bis 11, wobei der Reaktor ein Strömungsreaktor, beispielsweise ein Festbettreaktor, ein Rohrbündelreaktor, ein kontinuierlicher Rührkesselreaktor oder ein Schlaufenreaktor ist.

13. Verfahren nach einem der Ansprüche 1 bis 12, **dadurch gekennzeichnet, dass** der im Verfahren eingesetzte mindestens eine Reaktor adiabatisch, polytrop oder unter Verwendung eines Kühlmittels nahezu isotherm betrieben wird.

14. Verfahren nach einem der Ansprüche 1 bis 13, wobei die Oligomerisierung bei einer Temperatur zwischen 5 und 160 °C, besonders bevorzugt zwischen 30 und 110 °C, ganz besonders bevorzugt zwischen 40 und 90°C durchgeführt wird.

15. Verfahren nach einem der Ansprüche 1 bis 14, wobei die erste Reaktionsstufe mit einem Gesamtzulauf des isobutenhaltigen Einsatzstroms von mindestens 2,5 t pro t Katalysator und Stunde (h) (Einheit t/(t*h)), vorzugsweise mindestens 3 t/(t*h) und besonders bevorzugt mindestens 3,5 t/(t*h) befahren wird.

## Claims

1. Process for producing diisobutene by oligomerization of isobutene over an acid catalyst in at least one reaction stage which in each case contains at least one reactor and at least one distillation column, wherein
an isobutene-containing input stream which comprises an isobutene-containing hydrocarbon stream as feed and a recirculated stream as recycle is converted in the at least one reactor of the at least one reaction stage to obtain a product mixture containing at least the diisobutene formed having a proportion of 2,4,4-trimethylpent-1-ene of at least 75 mol%, preferably at least 76.5 mol%, particularly preferably at least 78 mol%, and unconverted isobutene, and the obtained product mixture is supplied to the at least one distillation column, at the top of the distillation column a residual hydrocarbon stream depleted in the diisobutene formed based on the obtained product mixture is withdrawn and at least partially recirculated to the at least one reactor as recycle, **characterized in that**
the ratio of recycle to feed is at least 4.

2. Process according to Claim 1, wherein the ratio of recycle to feed is at least 5.

3. Process according to Claim 2, wherein the ratio of recycle to feed is at least 6.

4. Process according to any of Claims 1 to 3, wherein the isobutene-containing hydrocarbon stream is a C4-hydrocarbon stream.

5. Process according to any of Claims 1 to 3, wherein the isobutene-containing hydrocarbon stream is a pure isobutene stream.

6. Process according to any of Claims 1 to 5, wherein the residual hydrocarbon stream withdrawn at the top of the column is completely recirculated to the at least one reactor.

7. Process according to any of Claims 1 to 6, wherein a reaction stage comprises at least two reactors which are connected in series or parallel relative to one another.

8. Process according to any of Claims 1 to 7, wherein the oligomerization is performed in the liquid phase.

9. Process according to any of Claims 1 to 8, wherein the acid catalyst is an acidic ion exchange resin in which a portion of the acidic protons have been exchanged for metal ions.

10. Process according to Claim 9, wherein 0.1% to 70% of the acidic protons, preferably 30% to 65% of the acidic protons, of the ion exchanger have been exchanged for metal ions.

11. Process according to Claim 9 or 10, wherein the metal ions are ions of the alkali metals, alkaline earth metals and/or rare earths.

12. Process according to any of Claims 1 to 11, wherein the reactor is a flow reactor, for example a fixed bed reactor, a tube bundle reactor, a continuous stirred tank reactor or a loop reactor.

13. Process according to any of Claims 1 to 12, **characterized in that** the at least one reactor employed in the process is operated adiabatically, polytropically or virtually isothermally using a coolant.

14. Process according to any of Claims 1 to 13, wherein the oligomerization is performed at a temperature between 5°C and 160°C, particularly preferably between 30°C and 110°C, very particularly preferably between 40°C and 90°C.

15. Process according to any of Claims 1 to 14, wherein the first reaction stage is run with a total feed of the isobutene-containing input stream of at least 2.5 t per t of catalyst and hour (h) (unit t/(t*h)), preferably at least 3 t/(t*h) and particularly preferably at least 3.5 t/(t*h).

## Revendications

1. Procédé de fabrication de diisobutène par oligomérisation d'isobutène sur un catalyseur acide dans au moins un étage de réaction, dont chacun comprend au moins un réacteur et au moins une colonne de distillation, dans lequel
un courant d'alimentation contenant du butène, qui comprend un courant d'hydrocarbures contenant de l'isobutène et servant de charge et un courant de retour servant de recyclage, est mis à réagir dans au moins un réacteur de l'au moins un étage de réaction avec obtention d'un mélange de produits qui contient au moins le diisobutène formé avec une proportion de 2,4,4-triméthylpent-1-ène d'au moins 75 % en moles, de préférence d'au moins 76,5 % en moles et d'une manière particulièrement préférée d'au moins 78 % en moles et de l'isobutène n'ayant pas réagi, et le mélange de produits obtenu est envoyé à l'au moins une colonne de distillation,
en tête de la colonne de distillation, un courant d'hydrocarbures résiduel épuisé en ledit isobutène formé, par rapport au mélange de produits obtenu, est soutiré et est renvoyé au moins partiellement sous forme d'un recyclage à l'au moins un réacteur, **caractérisé en ce que**
le taux de recyclage est d'au moins 4.

2. Procédé selon la revendication 1, dans lequel le taux de recyclage est d'au moins 5.

3. Procédé selon la revendication 2, dans lequel le taux de recyclage est d'au moins 6.

4. Procédé selon l'une des revendications 1 à 3, dans lequel le courant d'hydrocarbures contenant de l'isobutène est un courant d'hydrocarbures en C4.

5. Procédé selon l'une des revendications 1 à 3, dans lequel le courant d'hydrocarbures contenant de l'isobutène est un courant d'isobutène pur.

6. Procédé selon l'une des revendications 1 à 5, dans lequel le courant d'hydrocarbures résiduel soutiré en tête de la colonne est entièrement renvoyé à au moins un réacteur.

7. Procédé selon l'une des revendications 1 à 6, dans lequel un étage de réaction comprend au moins deux réacteurs, qui sont montés en série ou en parallèle les uns aux autres.

8. Procédé selon l'une des revendications 1 à 7, dans lequel l'oligomérisation a lieu en phase liquide.

9. Procédé selon l'une des revendications 1 à 8, dans lequel le catalyseur acide est une résine échange d'ions acide, dans laquelle une partie des protons acides ont été remplacés par des ions métalliques.

10. Procédé selon la revendication 9, dans lequel 0,1 à 70 % des protons acides, de préférence 30 à 65 % des protons acides de l'échangeur d'ions, sont remplacés par des ions métalliques.

11. Procédé selon la revendication 9 ou 10, dans lequel les ions métalliques sont des ions des métaux alcalins, des métaux alcalino-terreux et/ou des terres rares.

12. Procédé selon l'une des revendications 1 à 11, dans lequel le réacteur est un réacteur continu, par exemple un réacteur à lit fixe, un réacteur à faisceau tubulaire, un réacteur agitateur ou un réacteur à bulles à écoulement en boucle.

13. Procédé selon l'une des revendications 1 à 12, **caractérisé en ce que** l'au moins un réacteur utilisé dans le procédé est exploité dans des conditions adiabatiques, polytropiques ou par utilisation d'un réfrigérant dans des conditions presque isothermes.

14. Procédé selon l'une des revendications 1 à 13, dans lequel l'oligomérisation est mise en œuvre à une température entre 5 et 160 °C, d'une manière particulièrement préférée entre 30 et 110 °C, d'une manière tout particulièrement préférée entre 40 et 90 °C.

15. Procédé selon l'une des revendications 1 à 14, dans lequel le premier étage de réaction est exploité avec un débit total de courant d'alimentation contenant de l'isobutène d'au moins 2,5 t par t de catalyseur et par heure (h) (unité t/(t*h)), de préférence d'au moins 3 t/ (t*h) et d'une manière particulièrement préférée d'au moins 3,5 t/(t*h).
